(19)

Europäisches Patentamt
European Patent Office
Office européen des brevets

(11) **EP 2 380 975 A1**

(12) **EUROPÄISCHE PATENTANMELDUNG**

(43) Veröffentlichungstag:
**26.10.2011 Patentblatt 2011/43**

(51) Int Cl.:
*C12N 9/74* *(2006.01)* *C07K 1/113* *(2006.01)*

(21) Anmeldenummer: **10160740.6**

(22) Anmeldetag: **22.04.2010**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA ME RS**

(71) Anmelder: **Scil Proteins GmbH**
**06120 Halle/Saale (DE)**

(72) Erfinder:
• **Anton, Andreas**
  **06120 Halle (DE)**

• **Dietrich, Arndt**
  **06120 Halle (DE)**
• **Kötter, Jochen**
  **06120 Halle (DE)**
• **Schäffner, Jörg**
  **06120 Halle (DE)**

(74) Vertreter: **Sandmann, Wolfgang**
**Reinhard, Skuhra, Weise & Partner GbR**
**Patent- und Rechtsanwälte**
**Friedrichstrasse 31**
**80801 München (DE)**

(54) **Verfahren zur Herstellung von rekombinantem Thrombin**

(57) Gegenstand der Erfindung ist ein Verfahren zur Herstellung von gefaltetem Präthrombin, wobei Einschlusskörperchen, die nicht gefaltetes Präthrombin oder ein Derivat davon enthalten, in einem Solubilisierungspuffer solubilisiert werden, der mindestens eine chaotrope Verbindung und mindestens eine organische Disulfidverbindung enthält. Die Erfindung betrifft auch Verfahren zur Herstellung von Thrombin und α-Thrombin sowie Derivaten davon. Gegenstand der Erfindung sind auch Lösungen, die gefaltete Proteine enthalten, welche gemäß den erfindungsgemäßen Verfahren herstellbar sind.

EP 2 380 975 A1

**Beschreibung**

Gegenstand der Erfindung sind Verfahren zur Herstellung von gefaltetem Präthrombin und Thrombin aus Einschlusskörperchen. Gegenstand der Erfindung sind auch Lösungen, enthaltend gefaltete Proteine, die gemäß dem erfindungsgemäßen Verfahren herstellbar sind.

Stand der Technik

Die zentralen Enzyme der Blutgerinnungskaskade sind Serinproteasen, die in der Leber als etwas größere inaktive Vorstufen, sogenannte Zymogene, synthetisiert werden. Zu ihnen zählt Prothrombin, die inaktive Vorstufe von Thrombin, welches auch als Faktor II bezeichnet wird. Thrombin spielt eine zentrale Rolle in der Blutgerinnungskaskade und der Fibrinolyse. Die zentrale Funktion bei der Blutgerinnung macht Thrombin für die Anwendung in der humanen Medizin interessant. Es wird als Medikament seit langem angewendet, um beispielsweise Wunden möglichst schnell zu verschließen (Zymogenetics, Inc. "Annual Report on Form 10-K For the Year Ended December 31, 2003" 2003; Nakajima et al Ann. Thorac. Surg. 2005 79: 1793-1794).

Daneben ist Thrombin auch an vielen weiteren Prozessen beteiligt. So initiiert es zum Beispiel den Abbruch der Prothrombinaktivierung und den Start der Fibrinolyse (Esmon & Jackson Journal of Biological Chemistry. 1974 249 (24): 7791-7797). Des Weiteren haben verschiedene Untersuchungen gezeigt, dass Thrombin an der Embryogenese, der Kanzerogenese, der Regulierung des Gefäßdruckes, der Alzheimererkrankung und an Wundheilungsprozessen beteiligt ist (Grand et al Biochemical Journal." 1996 313(2): 353-368; Tsopanoglou & Maragoudakis Journal of Biological Chemistry. 1999 274(34): 23969-23976). Auf Grund seiner hohen Polyfunktionalität ist Thrombin ein sehr interessanter Wirkstoff für medizinische und biotechnologische Anwendungen. Es wird zur Spaltung von Fusionsproteinen und somit zur Entfernung von Reinigungs- und *Assaytags* eingesetzt (Jenny et al., Protein Expression and Purification, 2003 31 (1): 1-11.).

Thrombin wird aufgrund seiner Homologie zu anderen Proteasen wie Chymotrypsin, Trypsin und Elastase der Klasse der Serinproteasen zugeordnet. Den Serinproteasen ist ein gleicher Katalysemechanismus gemeinsam. Das aktive Zentrum der Serinproteasen ist durch die für sie typische katalytische Triade Serin-Histidin-Aspartat charakterisiert. Thrombin besteht aus zwei Ketten, die A-und die B-Kette, die über eine Disulfidbrücke miteinander verbunden sind. Die kürzere A-Kette des Thrombins zeigt keine Gemeinsamkeiten mit den Pankreasenzymen Trypsin, Chymotrypsin und Elastase. Die B-Kette dieser Serinproteasen, die drei weitere intramolekulare Disulfidbrücken aufweist, unterscheidet sich dagegen kaum (Bode et al. Protein Science. 1992 1(4): 426-471. Stubbs & Bode Thrombosis Research. 1993 69 (1): 1-58 Stubbs& Bode Trends Biochem Sci. 1995 Jan; 20(1):23-8. Review. Erratum in: Trends Biochem Sci 1995 Mar; 20(3):131). Wie alle Serinproteasen besteht auch das Thrombin im wesentlichem aus zwei sechsträngigen ß-Faltblättern, die jeweils zu einer fassartigen Struktur zusammengerollt sind. Die V-förmige A-Kette verläuft in einer durch die größere B-Kette ausgesparten Kerbe auf der dem aktiven Zentrum gegenüberliegenden Seite.

Das zugehörige Zymogen Prothrombin (72 kDa) besteht aus einer Gla-Domäne, 2 Kringeldomänen und einer A- und einer B-Kette (Hiller 2003, Dissertation zur Erlangung des Grades eines Doktors der Naturwissenschaften der Universität Bielefeld, Butenas & Mann Biochemistry, Moscow, Russian Federation, 2002, 67(1): 3-12). Die beiden A- und B-Ketten sind im aktiven Protein über eine Disulfidbrücke verbunden. Durch den Prothrombinasekomplex wird Prothrombin zwischen $Arg_{271}$-$Thr_{272}$ sowie zwischen $Arg_{320}$-$Ile_{321}$ gespalten. Dadurch wird der N-Terminus entfernt und die A- und B-Kette voneinander getrennt. Je nach Reihenfolge dieser Aktivierung entstehen unterschiedliche Zwischenprodukte, Meizothrombin und Präthrombin-2 (Rosing et al. Journal of Biological Chemistry, 1986 261: 4224-4228.).

Die Verwendung von rekombinantem Thrombin (*rh*-Thrombin) als Wirkstoff stellt eine sinnvolle Alternative zu den sich derzeit auf dem Markt befindlichen Produkten dar, welche aus bovinem und humanem Blutplasma gewonnen werden. Zum einen kann mit dem Einsatz von *rh*-Thrombin das Risiko der Übertragung von Infektionskrankheiten wie beispielsweise HIV und Hepatitis B auf ein Minimum reduziert werden. Zum anderen ist mit einer Immunantwort beim Menschen kaum zu rechnen, da es sich um einen Wirkstoff handelt, welcher sehr ähnliche Merkmale zum körpereigenen $\alpha$-Thrombin aufweist. Weiterhin ist bei der rekombinanten Herstellung mit einer erheblichen Kostenreduktion im Vergleich zu herkömmlichen Prozessen zu rechnen, da in Hochzelldichtefermentationsprozessen in der Regel sehr hohe Produktausbeuten erreicht werden können.

In der Literatur sind verschiedene Verfahren zur rekombinanten Herstellung von $\alpha$-Thrombin in eukaryontischen und prokaryotischen Zellen beschrieben. In den meisten Verfahren erfolgt die Gewinnung von $\alpha$-Thrombin aus dem inaktiven Vorläufermolekül Präthrombin-2, welches nach einem ersten chromatographischen Reinigungsschritt, durch die Metalloprotease Ecarin aktiviert wird (Russo et al., 1997, Protein Expr. Purif., 10(2):214-25; Yonemura et al., 2004, J. Biochem. (Tokyo), 135(5):577-82; Soejima et al., Journal of Biochemistry, 2001, 130(2): 269-277). Beispielsweise hat ZymoGenetics Inc. ein Herstellungsverfahren für *rh*-Thrombin in eukaryontischen CHO-Zellen entwickelt, wobei von Präthrombin-1 als Vorläufermolekül ausgegangen wird.

Die Herstellung von rekombinanten Proteinen in Säugerzellen erfordert in der Regel hohe Kosten, Zeit und liefert moderate Produktausbeuten. In den bekannten eukaryontischen Fermentationsprozessen werden Produktausbeuten von 25 bis 140 mg/l Fermentationsmedium erreicht. Das Zielprotein Präthrombin-2 wird dabei in das Medium sezerniert (Russo *et al.* 1997, Yonemura *et al.* 2004).

Zur Verbesserung dieser kritischen Parameter stellt die Herstellung in Prokaryoten eine Alternative dar.

Soejima *et a.,* 2001 offenbaren ein Verfahrung zur Expression von Präthrombin-2 in *Escherichia coli (E. coli)* mit anschließenden Schritten zur Solubilisierung und Faltung. Es werden nur geringe Produktausbeuten an gefaltetem nativem Präthrombin-2 erreicht. Im Vergleich zu den Ausbeuten, die in eukaryontischen Expressionssystemen erreicht werden, erweist sich der von Soejima *et al.* 2001 beschriebene Prozess als nachteilig.

Aufgabe der Erfindung

Der Erfindung liegt die Aufgabe zugrunde, ein Verfahren zur Herstellung von gefaltetem Präthrombin und Thrombin bereitzustellen, das die oben beschriebenen Nachteile überwindet.

Der Erfindung liegt insbesondere die Aufgabe zugrunde, ein Verfahren zur Herstellung von Thrombin und Präthrombin zu entwickeln, das einfach durchführbar ist und eine hohe Ausbeute erzielt.

Dabei soll es das Verfahren ermöglichen, gefaltetes Präthrombin und Thrombin in hohen Ausbeuten und in hoher Reinheit herzustellen. Das Verfahren soll bevorzugt ein prokaryotisches Expressionssystem nutzen.

Gegenstand der Erfindung:

Überraschenderweise wird die Aufgabe gelöst durch Verfahren gemäß den Patentansprüchen.

Gegenstand der Erfindung ist ein Verfahren zur Herstellung von gefaltetem Präthrombin oder einem Derivat davon, wobei Einschlusskörperchen, die nicht gefaltetes Präthrombin oder ein Derivat davon enthalten, in einem Solubilisierungspuffer solubilisiert werden, der mindestens eine chaotrope Verbindung und mindestens eine organische Disulfidverbindung enthält.

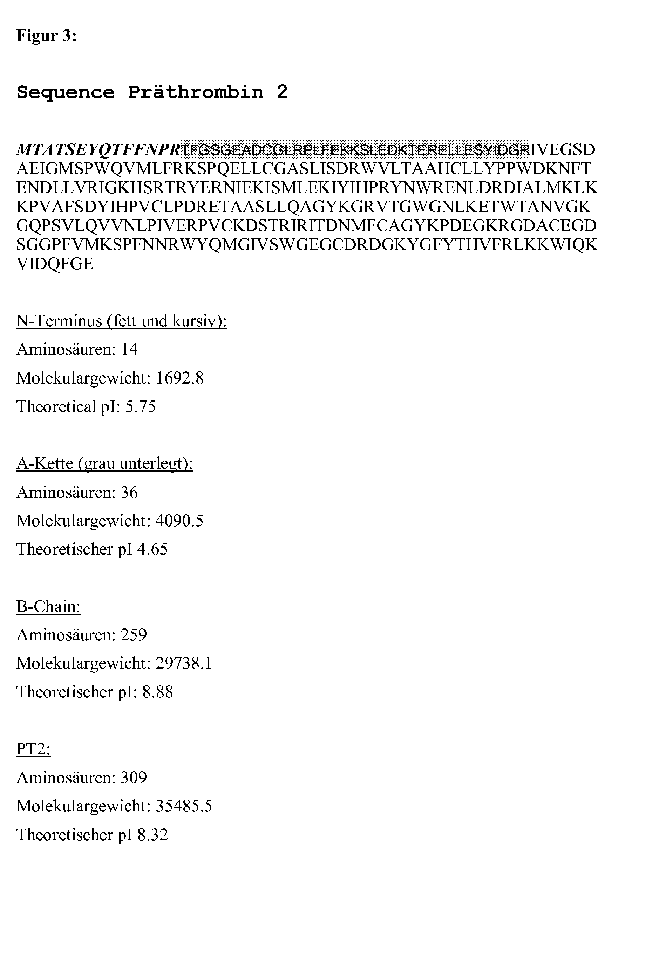

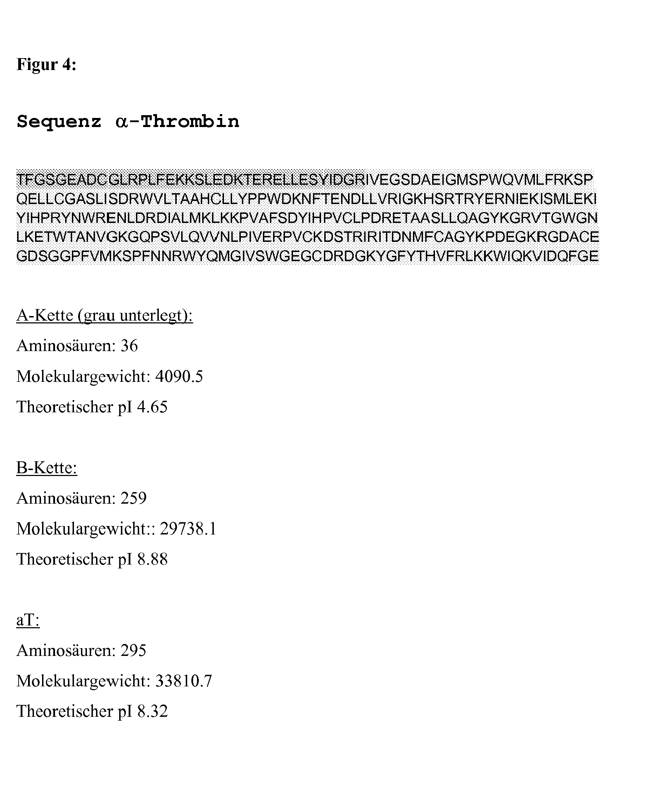

Das erfindungsgemäße Verfahren dient zur Herstellung und Reinigung von menschlichem Präthrombin. Bevorzugt wird Präthrombin-2 eingesetzt. Das Protein weist 309 Aminosäuren und ein Molekulargewicht von 35485,5 Da auf. Die Aminosäuresequenz ist in Figur 3 gezeigt. Das humane Präthrombin kann zu $\alpha$-Thrombin gespalten werden, das 295 AS und ein Molekulargewicht von 33810,7 Da aufweist. Die Sequenz und die A- und B-Kette sind in Figur 4 gezeigt.

Erfindungsgemäß können auch Derivate von Präthrombin eingesetzt werden. Dabei handelt es sich um Derivate, bei denen die prozessierte Form eine thrombinähnliche Aktivität, insbesondere Proteaseaktivität, aufweisen. Die Derivate von Präthrombin sind entsprechende Vorläufer-Polypeptide, aus denen durch proteolytische Spaltung die aktiven Thrombin-Derivate erhalten werden können. Die Derivate sind insbesondere solche, die durch Aminosäuremutation, -deletion oder - insertion erhalten werden können, oder durch chemische Modifizierung des Polypeptids. Bevorzugt weisen die Derivate eine Sequenzidentität zum Wildtyp-Protein von mehr als 80%, mehr als 90% oder mehr als 95% auf. Die Derivate weisen mindestens ein Cystein pro Polypeptidkette auf, bevorzugt jedoch alle nativen Cysteine. Für den Fachmann ist ersichtlich, dass das erfindungsgemäße Verfahren allgemein auch mit Derivaten von Präthrombin durchführbar ist. Nach der Faltung und Abspaltung von Präpeptiden werden die entsprechenden Derivate von Thrombin oder $\alpha$-Thrombin erhalten. Daher betreffen Ausführungen, die nachfolgend zu dem Verfahren gemacht werden und bei denen auf Präthrombin, Thrombin und $\alpha$-Thrombin Bezug genommen wird, gleichermaßen die Derivate dieser Polypeptide. Bei den nachfolgenden allgemeinen Erläuterungen kann also der Begriff "Präthrombin" mit "Präthrombin oder Derivaten von Präthrombin" gleichgesetzt werden. Dies gilt gleichermaßen für Thrombin und $\alpha$-Thrombin.

Bei dem erfindungsgemäßen Verfahren werden Einschlusskörperchen solubilisiert. Einschlusskörperchen (Inclusion Bodies, IB) sind Ansammlungen von zumeist fehlerhaft oder unvollständig gefalteten Proteinen. Sie bilden sich im Inneren von Zellen, beispielsweise Bakterienzellen, wie *E. coli,* bei übermäßiger Expression rekombinanter Proteine. Bevorzugt enthalten die erfindungsgemäß eingesetzten Einschlusskörperchen das gefaltete Präthrombin in hoher Reinheit. Dies bedeutet, dass sie mindestens 60, mindestens 70, mindestens 80 oder mindestens 90 Gew.-% Präthrombin (bezogen auf die Gesamtmenge Protein) aufweisen.

In dem Solubilisierungspuffer ist eine Disulfidverbindung enthalten. Die Disulfidverbindung ist in der Lage, mit Thiolgruppen (-SH) von Cysteinen der Polypeptide in den Einschlusskörperchen gemischte Disulfide zu bilden. Das Disulfid wird der Lösung zugesetzt. Mit dem Disulfid werden nicht Proteine bezeichnet, die in den Einschlusskörpern enthalten sind und die möglicherweise Disulfidbrücken enthalten. Bevorzugt ist das Disulfid nicht ein echtes Peptid. Bevorzugt ist das Disulfid eine niedermolekulare Verbindung. Das Molekulargewicht ist beispielsweise niedriger als 2000 g/Mol oder als 1000 g/Mol. Das Disulfid wird beispielsweise in einer Konzentration von 5 mM bis 1 M, insbesondere 10 mM bis 0,5 M, eingesetzt.

In einer bevorzugten Ausführungsform der Erfindung ist die Disulfidverbindung Glutathion-Disulfid. Glutathion (GSH), auch γ-L-Glutamyl-L-cysteinylglycin, ist ein Pseudotripeptid, das aus den drei Aminosäuren Glutaminsäure, Cystein und Glycin gebildet wird. GSH ist im Cytoplasma von sowohl Prokaryoten als auch Eukaryoten vorhanden und an der Ausbildung von Disulfidbrücken beteiligt. Es liegt in einem Gleichgewicht mit dem Dimer GSSG vor, das eine Disulfidbrücke aufweist. Glutathion reagiert in einer Disulfidaustauschreaktion mit Cysteinen R-SH und R'-SH von zwei Polypeptiden oder von einem einzigen Polypeptid:

$$R\text{-SH} + GSSG \rightarrow R\text{-S-S-G} + GSH.$$

Das RSSG bezeichnet man als gemischtes Disulfid. Es wird mit einem weiteren Cystein eines Polypeptides umgesetzt, so dass im Ergebnis eine Disulfidbrücke zwischen zwei Cysteinen erhalten wird:

$$R\text{-S-S-G} + HS\text{-R'} \rightarrow R\text{-S-S-R'} + GSH.$$

Im Cytosol wird Glutathion enzymatisch in reduzierter Form gehalten (GSH). Man spricht daher von "reduzierenden Bedingungen" im Cytosol. In dem Solubilisierungspuffer werden Bedingungen eingestellt, so dass die enthaltene Disulfidverbindung die Ausbildung von Disulfidbrücken gemäß den oben beschriebenen Reaktionen katalysiert. Das GSSG wird beispielsweise in einer Konzentration von 10 mM bis 0,5 M eingesetzt.

In dem Solubilisierungspuffer ist mindestens eine chaotrope Substanz enthalten. Als chaotrop werden chemische Substanzen bezeichnet, die geordnete Wasserstoffbrückenbindungen in Wasser auflösen. Indem die Wasserstoffbrückenbindungen aufgebrochen werden, stören die chaotropen Substanzen die Wasserstruktur und sorgen für Unordnung (Zunahme der Entropie). Die Ursache dessen ist, dass die Bildung der zur Solvatisierung notwendigen $H_2O$-Käfigstrukturen verhindert wird. Bei Aminosäuren vermindern sie hydrophobe Effekte und wirken denaturierend auf Proteine, da eine treibende Kraft der Proteinfaltung die Zusammenlagerung der hydrophoben Aminosäuren im Wasser ist. Allgemein ist als chaotrope Substanz jede Substanz einsetzbar, die in dem Solubilisierungspuffer den hydrophoben Effekt ausübt und somit auf die Proteine denaturierend wirkt. Chaotrope Substanzen sind im allgemeinen Salze oder niedermolekulare Verbindungen wie Harnstoff. Chaotrope Substanzen sind deutlich von Detergentien unterschieden, da sie keinen hydrophoben Rest, wie einen Alkylrest, im Molekül aufweisen. Allgemein geht die chaotrope Wirkung mit der Verbesserung der Löslichkeit des Proteins, hier des Präthrombins, einher.

In einer bevorzugten Ausführungsform der Erfindung ist die chaotrope Verbindung ausgewählt aus Guanidiniumsalzen, insbesondere Guanidiniumhydrochlorid und Guanidiniumthiocyanat, Jodiden, Bariumsalzen, Thiocyanaten, Harnstoff und Perchloraten.

Die chaotropen Verbindungen werden in üblichen Mengen eingesetzt. Beispielsweise können 4-8 M Guanidiniumhydrochlorid oder 4-9 M Harnstoff eingesetzt werden.

In einer bevorzugten Ausführungsform der Erfindung ist der Solubilisierungspuffer ein Tris-Puffer. In einer bevorzugten Ausführungsform der Erfindung ist in das Reagens, welches mit den -SH Gruppen des Proteins in den Einschlusskörperchen gemischte Disulfide bilden kann, GSSG und die chaotrope Substanz ist Guanidiniumhydrochlorid.

Der Solubilisierungspuffer kann weitere übliche Zusätze enthalten, beispielsweise EDTA oder Salze. Der pH-Wert des Solubilisierungspuffers liegt beispielsweise zwischen 6 und 11, bevorzugt zwischen 7 und 10. Die Solubilisierung wird bevorzugt mechanisch unterstützt, beispielsweise mit üblichen Homogenisierungsgeräten oder mittels Ultraschall. Nach dem Solubilisieren werden bevorzugt verbliebene Feststoffe abgetrennt. In dem Überstand ist das solubilisierte Präthrombin enthalten.

In einer bevorzugten Ausführungsform der Erfindung wurden die Einschlusskörperchen durch rekombinante Expression von Präthrombin in prokaryotischen Zellen erhalten. In einer bevorzugten Ausführungsform der Erfindung ist die prokaryotische Wirtszelle ein Bakterium, bevorzugt *E. coli*. In einer bevorzugten Ausführungsform der Erfindung ist das Bakterium *E. coli* JM108 (DSMZ 5585; 3.3.2006), bevorzugt transformiert mit einem aus pSCIL008 hervorgehenden Plasmid.

Bevorzugt wird nach der Solubilisierung überschüssige Disulfidverbindung entfernt. Die Entfernung des Reagens erfolgt bevorzugt mit einer Methode zum Pufferaustausch, also über Dialyse, Chromatographie oder Tangential Fluss Filtration.

In einer bevorzugten Ausführungsform der Erfindung wird das solubilisierte Präthrombin in einem Renaturierungspuffer renaturiert, der mindestens ein Reduktionsmittel, mindestens einen Faltungshelfer und divalente Kationen aufweist. In einer bevorzugten Ausführungsform der Erfindung wird das Solubilisat in mehreren Fraktionen oder kontinuierlich über mehrere Tage zum Faltungsansatz gegeben. Bevorzugt wird das Solubilisat in einer "Pulsrenaturierung" durch schnelles Verdünnen zu dem Solubilisat gegeben. Dabei können beispielsweise 6 Pulse im zeitlichen Abstand von 24 Stunden durchgeführt werden. Die Zahl der Pulse wird so eingestellt, dass die Konzentration an noch nicht gefaltetem Protein nach der Zugabe des Solubilisierungsansatzes nicht zu hoch wird, da sonst Aggregate erhalten werden. Beispielsweise werden mit jedem Puls etwa 0,1 g/l Protein neu in den Faltungsansatz überführt (bezogen auf die Proteinkonzentration im Faltungsansatz nach Zugabe des Solubilisats). So kann im Faltungsansatz eine Proteinendkonzentration von 0,6 g/l erreicht werden.

In einer bevorzugten Ausführungsform der Erfindung ist das Reduktionsmittel ein organisches Monosulfid. Bevorzugt ist dabei der Einsatz von Glutathion (GSH). Es kann auch ein Gemisch aus GSH/GSSG eingesetzt werden.

In einer bevorzugten Ausführungsform der Erfindung ist der Faltungshelfer ausgewählt aus Arginin und Glycerin. Als "Faltungshelfer" sind allgemein Verbindungen einsetzbar, welche die Faltung von Proteinen fördern. Solche Verbindungen sind dem Fachmann bekannt. Sie können die Faltung auf unterschiedliche Weise unterstützen. Von Arginin wird angenommen, dass es falsch gefaltete Intermediate destabilisiert, so dass diese wieder (aus einer thermodynamischen Sackgasse) zumindest teilweise entfaltet werden und damit wieder richtig faltbar sind. Glycerin stabilisiert dagegen normalerweise Proteine. Als Faltungshelfer sind insbesondere Verbindungen geeignet, die bei dem erfindungsgemäßen Verfahren die absolute Ausbeute von gefaltetem Präthrombin um mehr als 5%, insbesondere um mehr als 10 oder um mehr als 20% (bezogen auf die Gesamtmenge des zur Faltung eingesetztes Präthrombins) erhöhen, verglichen mit einem Verfahren ohne Einsatz des Faltungshelfers.

Der Renaturierungspuffer enthält divalente Kationen. Bevorzugt ist der Einsatz von Calciumsalzen, insbesondere Calciumchlorid. Der Renaturierungspuffer ist bevorzugt ein Tris-Puffer.

Die Renaturierung erfolgt bevorzugt bei einem pH-Wert zwischen 6 und 12, insbesondere zwischen 7 und 11.

In einer bevorzugten Ausführungsform der Erfindung weist der Solubilisierungspuffer und/oder der Renaturierungspuffer kein Detergens auf. Es wurde erfindungsgemäß gefunden, dass der Einsatz von Detergenzien zur Solubilisierung und/oder Faltung von Präthrombin nicht erforderlich ist. Dies ist vorteilhaft, da bestimmte Detergenzien vergleichsweise aggressive chemische Substanzen sind, die in pharmazeutischen Produkten nicht oder nur in geringen Mengen enthalten sein dürfen und somit aufwändig entfernt werden müssen. Das erfindungsgemäße Verfahren ist daher vorteilhaft gegenüber dem Verfahren von Soejima et. al., 2001, bei dem zur Faltung des Proteins solche aggressiven Detergenzien (Triton X-100 oder Brij-58) eingesetzt werden.

Mit dem erfindungsgemäßen Verfahren werden überraschend hohe Renaturierungsausbeuten erreicht. Beispielsweise konnten Renaturierungsausbeuten bis zu 25%, bezogen auf das gesamte Präthrombin in den Einschlusskörperchen, erreicht werden. Bevorzugt wird eine Renaturierungsausbeute von mindestens 10% oder mindestens 20% erreicht. Die Proteinendkonzentration kann beispielsweise bei 0,6 g/l liegen. Sie liegt bevorzugt zwischen 0,1 und 2 g/l, insbesondere höher als 0,3 g/l, um große Faltungsvolumen in der Produktion zu vermeiden.

Bei Durchführung des erfindungsgemäßen Verfahrens mit der Solubilisierung und anschließenden Renaturierung wird eine wässrige Lösung von gefaltetem Präthrombin erhalten. Das gefaltete Präthrombin kann anschließend durch bekannte Verfahren weiter aufgereinigt werden.

Erfindungsgemäß wurde gefunden, dass für die weitere Aufreinigung eine chromatographische Reinigung, insbesondere mittels hydrophober InteraktionsChromatographie, besonders vorteilhaft ist. Als Säule kann beispielsweise eine Phenylsepharose HP von GE Healthcare eingesetzt werden. Die HIC-Chromatographie erfolgt bevorzugt in Gegenwart von Ammoniumsulfat, bevorzugt bei einer Konzentration von 1,15 M im Hochsalzpuffer. Es wurde gefunden, dass unter diesen Bedingungen Produktausbeuten von 70 bis 95% bei einer Elution mittels Stufengradient erzielt werden können.

Bevorzugt erfolgt die Reinigung nicht durch Hirudin-basierte Affinitätschromatographie. Bevorzugt erfolgt bei dem erfindungsgemäßen Verfahren kein Reinigungsschritt mit Hirudin-basierter Affinitätschromatographie.

Bevorzugt wird das renaturierte Präthrombin durch enzymatische Proteolyse, insbesondere mit einer Small Venom Protease, bevorzugt Ecarin, zu Thrombin umgesetzt. Die Serinprotease Ecarin spaltet die Peptidbindung zwischen der A- und B-Kette an der Position Arginin 320 und Isoleucin 321 von Präthrombin-2. Aus dem so entstandenen Thrombin wird durch autokatalytische Abspaltung des eigenen N-Terminus α-Thrombin. Bei dem erfindungsgemäßen Verfahren wird also bei ausreichender Inkubationszeit Thrombin als Zwischenprodukt und α-Thrombin als Endprodukt erhalten. Bei nicht vollständiger autokatalytischer Spaltung wird ein Gemisch von Thrombin und α-Thrombin erhalten. Nachfolgend steht der Begriff "Thrombin" auch für dieses Gemisch. Zur proteolytischen Spaltung können auch Proteasen eingesetzt werden, die eine ähnliche Funktionalität wie Ecarin aufweisen. Bevorzugt wird die Protease in einer Konzentration von weniger als 2 U/ml, bevorzugt weniger als 1 U/ml oder weniger als 0,5 U/ml eingesetzt.

Bevorzugt wird das Thrombin und/oder α-Thrombin nach der Proteolyse chromatographisch gereinigt. Bevorzugt erfolgt die Reinigung durch Hydrophobe Interaktions-Chromatographie (HIC). Als kritisch hat sich während der Prozessführung die durch α-Thrombin verursachte Ausbildung von Autolyseprodukten erwiesen, welche nach Aktivierung von Präthrombin-2 spontan bei längerer Lagerung entstehen können. Die Abreicherung dieser produktbezogenen Verunreinigungen mittels Ionenaustauscherchromatographie (IEX) erwies sich als schwierig, da die pI-Werte von α-Thrombin und seiner Autolyseprodukte nahezu identisch sind. Verwendete man stattdessen eine Hydrophobe Interaktionschromatographie (HIC), so konnten deutlich günstigere Abreicherungseffekte beobachtet werden. Somit ist es erfindungsgemäß bevorzugt, das gefaltete Präthrombin nach der Renaturierung und das Thrombin nach der proteolytischen Spaltung des Präthrombins mittels HIC zu reinigen. Allgemein bietet die Aufreinigung von gespaltetem Präthrombin in wässriger Lösung oder von Thrombin bzw. α-Thrombin in wässriger Lösung durch HIC Vorteile, die nicht auf das vorliegende spezielle Verfahren zur Herstellung von gefaltetem Präthrombin aus Einschlusskörpern mit den Merkmalen von Anspruch 1 beschränkt sind. Gegenstand der Erfindung ist daher auch allgemein die Reinigung von wässrigen Lösungen, enthaltend gefaltetes Präthrombin oder Thrombin bzw. α-Thrombin mittels HIC.

In einer bevorzugten Ausführungsform der Erfindung wird neben der Reinigung des erhaltenen Thrombins mittels HIC ein weiterer chromatographischer Reinigungsschritt durchgeführt. Vorzugsweise wird Ionenaustauscherchromatographie (IEX) eingesetzt. Dadurch kann der Gehalt an Endotoxinen, an DNA und an *Host cell* Proteinen (HCP) auf ein Minimum reduziert werden.

Am Ende der Prozessführung könnte eine Diafiltration mit sich anschließender Konzentrierung des Wirkstoffes stehen. Es können jedoch auch andere bekannte Methoden eingesetzt werden, um die Reinheit und Konzentration des Thrombins zusätzlich zu erhöhen.

Untersuchungen zur Lagerstabilität von α-Thrombin haben gezeigt, dass die Zugabe von Aminosäuren und Salz die Autolyse unterdrücken und gleichzeitig die Stabilität von α-Thrombin über mindestens 3 Monate bei 4°C gewährleisten.

In einer bevorzugten Ausführungsform der Erfindung umfasst das erfindungsgemäße Verfahren die folgenden Schritte:

a) Expression von rekombinantem Präthrombin in prokaryotischen Zellen und Isolierung der Präthrombin-haltigen Einschlusskörperchen

b) Mischung der Einschlusskörperchen mit einem geeigneten Solubilisierungspuffer, enthaltend zumindest eine chaotrope Substanz und eine Disulfidverbindung, die mit den -SH Gruppen des Proteins in den Einschlusskörperchen gemischte Disulfide bilden kann,

c) Entfernung von überschüssigem Disulfid,

d) Renaturierung in einem geeigneten Detergenz-freien Puffer, enthaltend mindestens ein Reduktionsmittel, einen Faltungshelfer und divalente Kationen,

e) Reinigung des renaturierten Präthrombins,

f) Spaltung in die aktive Form und

g) Isolierung und Reinigung des Thrombins und/oder α-Thrombins.

In einer bevorzugten Ausführungsform der Erfindung erfolgt in Schritt b) die Mischung der Einschlusskörperchen im Verhältnis von 1:4 bis 1:19 (1 g IB Paste+ 4 ml Solubilisierungspuffer) mit einem geeigneten Puffer mit pH im neutralbasischem Bereich, enthaltend 10 mM - 0,5 M GS S G, 4-8 M Guanidiniumhydrochlorid (GuaHCl) und 0,1-10 mM EDTA.

In einer bevorzugten Ausführungsform der Erfindung ist in Schritt b) die Mischung der Einschlusskörperchen im Verhältnis von 1:9 mit einem geeigneten Puffer mit pH im neutral-leicht basischem Bereich, enthaltend 0,1 M GSSG, 5 M Guanidin-hydrochlorid (GuaHCl) und 1 mM EDTA.

In einer bevorzugten Ausführungsform der Erfindung wird in Schritt c) die Entfernung von überschüssigem Reagens zur Bildung von gemischten Disulfiden durch Pufferwechsel in 3-8 M, bevorzugt 5 M, Guanidin-hydrochlorid bei saurem pH (pH 3,0) erreicht.

In einer bevorzugten Ausführungsform der Erfindung wird die Pulsrenaturierung in Schritt d) mit 0,01 - 1,0 g/l Protein (bezogen auf die Proteinkonzentration im Faltungsansatz pro Puls) in einem geeigneten Detergenz-freien Puffer mit neutral-leicht basischem pH, enthaltend 0,1-3 mM GSH, 0,1-2 M Arginin, 0,001 - 1M $CaCl_2$, 0,1-50 mM EDTA und 1-40% Glycerin, durchgeführt.

In einer bevorzugten Ausführungsform der Erfindung wird Schritt d) durch schnelles Verdünnen von 0,1 g/l Solubilisat in einem geeigneten Detergenz-freien Puffer mit neutral-leicht basischem pH, enthaltend 0,75 mM GSH, 1 M Arginin, 50 mM $CaCl_2$, 1 mM EDTA und 20 % Glycerin, durchgeführt, wobei die Proteinkonzentration im Faltungsansatz pro Puls um 0,1 g/l steigt und 6 Pulse mit einem Abstand von jeweils 24 h durchgeführt werden.

Die vorliegende Erfindung beschreibt ein neuartiges prokaryotisches Verfahren zur Herstellung von Thrombin im industriellen Maßstab, bei dem die Ausbeute an Präthrombin um ein Vielfaches erhöht ist und somit den Aufwand und die Kosten gegenüber bekannten Verfahren deutlich verringert. Die erhöhte Ausbeute wird insbesondere durch neue Solubilisierungs- und Renaturierungsverfahren erreicht.

Figuren:

Figur 1: zeigt die Reinheit von Präthrombin-2 nach der Renaturierung im SDS-PAGE Gel. Die Renaturierung von Präthrombin-2 erfolgte im Pulsrenaturierungsverfahren bis zu einer Proteinendkonzentration von 0,6 g/l. Es wurden Renaturierungsausbeuten bis zu 25 % erreicht. Dargestellt ist der Renaturierungsansatz im Coomassie gefärbten SDS-PAGE Gel.

Figur 2: zeigt die Reinigung von $\alpha$-Thrombin über Hydrophobe InteraktionsChromatographie (Toyopearl Butyl-650S). Die filtrierte Ecarinspaltung (4,5 ml + 4,5 ml Hochsalzpuffer: 2 M Ammoniumsulphat, 50 mM Phosphatpuffer pH 6,0) wurden über eine 4 ml HIC Säule (Tricorn 5/200) gereinigt. Die Äquilibrierung erfolgte mit 50 % Hochsalzpuffer und die Elution mittels linearem Gradienten über 20 CV auf 50 mM Phosphatpuffer pH 6,0.

Figur 3: zeigt die Aminosäuresequenz, die Struktur und wichtige Eigenschaften von Präthrombin-2.

Figur 4: zeigt die Aminosäuresequenz, die Struktur und wichtige Eigenschaften von alpha-Thrombin.

Ausführungsbeispiele

Beispiel 1: Expression von *rh*-Präthrombin-2

Der bakterielle Wirt *E. coli* JM108, der zur Expression von *rh*-Präthrombin-2 verwendet wird (DSMZ 5585; *F⁻ thi Δ (lac-proAB) end A*1 *gyrA96 relA1 phx hsdR17 supE44 recA),* ist Prolin-auxotroph, was durch den Einsatz von dem Plasmid mit der Bezeichnung pSCIL048 aufgehoben wird. Das Plasmid pSCIL048 basiert auf dem Plasmid pSCIL008 (siehe W005061716). Der Stamm kann kein Thiamin synthetisieren (Vieira & Messing, 1982 Gene. Oct; 19(3):259-68). Präthrombin-2 wird unter Kontrolle des *tac*-Promotors, der auf pSCIL048 lokalisiert ist, exprimiert. Der hier verwendete Vektor pSCIL048 ist ein *high copy* Plasmid mit einer Kanamycin Resistenz. Die Expression erfolgt in definiertem Mineralsalzmedium und wird durch die Zugabe von IPTG induziert. Das Präthrombin-2 wird in Form von Einschlusskörpern (IBs) im Cytosol abgelagert.

Die Biomasseproduktion erfolgte bei 37°C. Das Ziel dieser Fermentation bestand in der Gewinnung von Produkt und Biomasse für nachfolgende Prozessschritte. Zur Kontrolle der Überexpression des Zielproteins während des Fermentationsprozesses wurden Proben vor und nach Induktion mittels SDS-PAGE analysiert. Eine biomassespezifische Zunahme der Proteinkonzentration konnte bis zu 3 h nach Induktion beobachtet werden.

Beispiel 2: Zellaufschluss und Einschlusskörperchen *(IB, Inclusion Bodies)* Präparation

Die Expression des Zielproteins Präthrombin-2 erfolgte in Form von *IBs.* Der Zellaufschluss sowie die *IB* Präparation erfolgten nach Standardprotokollen und sind im Labormaßstab bis zu einer Aufarbeitung von ca. 200 g Biomasse durchführbar.

Beispiel 3: Solubilisierung und Renaturierung

Im erfindungsgemäßen optimierten Renaturierungsprotokoll erfolgte die Rückfaltung auf Basis von gemischten Disulfiden.

Zur Herstellung der gemischten Disulfide wurden die *IBs* in einem Verhältnis von 1 g IB Paste + 9 ml Solubilisierungspuffer mit 5 M GuaHCl; 0,1 M Tris-HCl; 1 mM EDTA; 0,1 M GSSG pH 8,5 homogenisiert und für 3 h bei RT solubilisiert. Nach einem Zentrifugationsschritt bei 50.000 x g über 30 min erfolgte ein Umpufferungsschritt in 5 M GuaHCl, 1 mM HCl pH 3,0 zur Abtrennung von freiem GSSG- / GSH-Gemisch.

Nach einem Zentrifugationsschritt bei 50.000 x g über 30 min (optional) erfolgte ein Umpufferungsschritt in 5 M GuaHCl (3-8 M), 1 mM HCl pH 3,0 (saurer pH ist wichtig, wenn das Solubilisat nicht direkt im Anschluss in den Faltungsansatz gegeben wird) zur Abtrennung von freiem GSSG- / GSH-Gemisch.

Die Pulsrenaturierung erfolgte durch schnelles Verdünnen des Solubilisates in dem Faltungspuffer 1 M Arginin, 50 mM Tris, 50 mM CaCl$_2$, 1 mM EDTA, 20 % Glycerin, 0,75 mM GSH pH 8,5, wobei bevorzugt bis zu 6 Pulse in einem zeitlichen Abstand von 24 h gesetzt wurden. Je Puls wurden 0,1 g/l Protein neu in den Faltungsansatz überführt (bezogen auf die Proteinkonzentration im Faltungsansatz nach Zugabe des Solubilisats). Im Faltungstank wurde eine Proteinendkonzentration von 0,6 g/l erreicht.

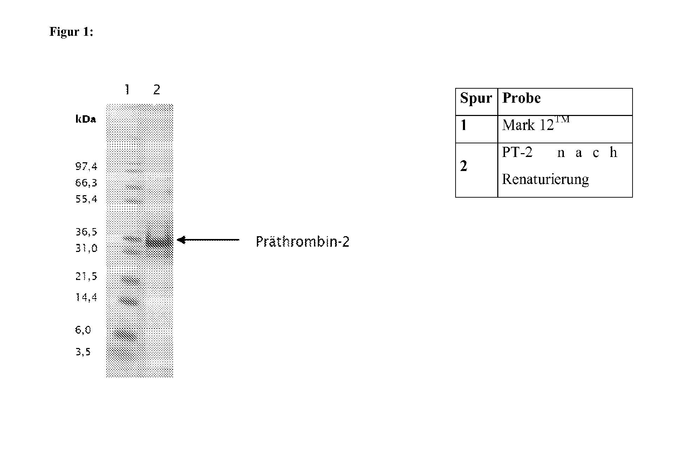

In Figur 1 ist die Reinheit des Produktes nach der Renaturierung dargestellt. Mit diesem Verfahren wurden Renaturierungsausbeuten von 25 %, bezogen auf die in den Faltungsansatz eingetragene Menge an Solubilisat, erreicht.

Beispiel 4: Reinigung von Präthrombin-2

Da bei Austausch mittels Diafiltration relativ hohe Ausbeuteverluste auftraten, wurde stattdessen Ammoniumsulfat zugegeben (bevorzugt 1,15 M Endkonzentration) Der Fällungsniederschlag wurde mittels Zentrifugation abgetrennt und der Überstand auf eine HIC-Säule aufgetragen. (bevorzugt Phenyl Sepharose HP,GE Healthcare), um Präthrombin dann im gewünschten Puffer zu eluieren.

Beispiel 5: Aktivierung von Präthrombin

Die Aktivierung von Präthrombin-2 zu $\alpha$-Thrombin erfolgte mit der Serinprotease Ecarin, welche spezifisch die Peptidbindung zwischen A und B Kette ($Arg_{320}$-$Ile_{321}$) des Präthrombin-2 spaltet. Aus dem so entstandenen Thrombin wird durch autokatalytische Abspaltung des eigenen N-Terminus $\alpha$-Thrombin. Die Spaltungsbedingungen wurden so gewählt, dass eine maximale Spaltungsausbeute bei möglichst geringem Ecarinbedarf erreicht wird. Dabei wurden mit 1 U Ecarin 2000 $\mu$g Präthrombin-2 (0,5 - 0,8 mg/ml) in 24 h bei 37 °C und 600 rpm im Schüttelinkubator gespalten. Die Reaktion wurde durch Zugabe von EDTA auf eine Endkonzentration von 25 mM abgestoppt. Nach der Spaltung wurde präzipitiertes Protein beobachtet, deshalb wurde die Lösung vor der chromatographischen Aufreinigung filtriert (0,2 $\mu$m). Die Spaltungsausbeute lag, je nach Menge des Präzipitates, zwischen 70 % und 90 %.

Beispiel 6: Reinigung von $\alpha$-Thrombin

Als Ausgangsmaterial für die chromatographische Aufreinigung wurde die filtrierte Ecarinspaltung verwendet. Das Ziel der Aufreinigung war neben der Abtrennung von Wirtszellproteinen vor allem die Abreicherung von ungespaltenem Präthrombin-2 und der autokatalytischen Spaltprodukte von $\alpha$-Thrombin. Präthrombin-2 liegt zum Zeitpunkt der Ecarinspaltung in einer Reinheit von mehr als 95 % vor. Um die Abreicherung der Spaltprodukte besser untersuchen zu können, wurden diese durch Inkubation der Spaltungsansätze für weitere 24 h bei 37 °C angereichert.

Die Abtrennung der Thrombinderivate mittels Ionenaustauschchromatographie gestaltete sich schwierig, da sie nahezu identische pI Werte (pI~9) aufweisen. Die pI-Werte von $\alpha$-Thrombin und seiner Autolyseprodukte wurden mittels 2D-Gelelektrophorese experimentell bestimmt. Neben Kationenaustauschmaterialien wurden daher hydrophobe Interaktionschromatographie-Materialien getestet.

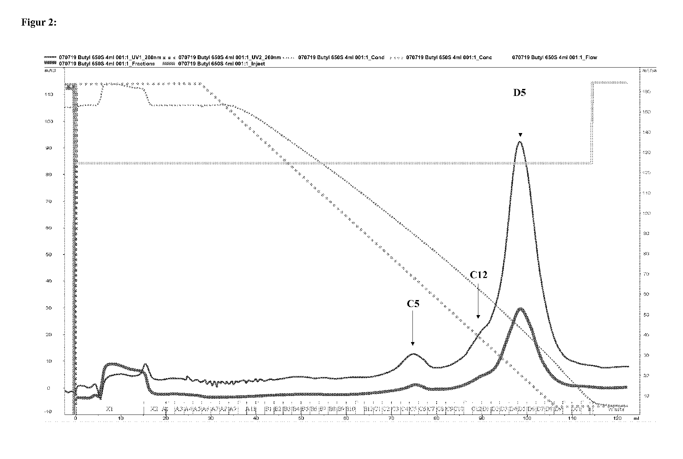

In Figur 2 ist das Chromatogramm einer Aufreinigung über eine 4 ml HIC Säule (Toyopearl Butyl-650S) gezeigt. Dazu wurden 4,5 ml der filtrierten Ecarinspaltung vor dem Auftrag auf die Säule 1:1 mit Hochsalzpuffer (2 M Ammoniumsulphat, 50 mM Phosphatpuffer pH 6.0) verdünnt. Die Elution erfolgte mittels eines linearen Gradienten (Start mit 50 % Hochsalzpuffer) auf 50 mM Phosphatpuffer pH 6.0 über 20 CV. Um das Ergebnis der Aufreinigung zu untersuchen, wurde direkt nach der Chromatographie zu den Hauptfraktionen PMSF bis zu einer Endkonzentration von 1 mM zugegeben, um eine weitere Autolyse zu inhibieren. Insgesamt liegt die Ausbeute an $\alpha$-Thrombin im Hauptpeak bei 65 % (bestimmt mit $UV_{280}$).

Ergebnis:

Bei dem erfindungsgemäßen Verfahren zur Herstellung von *rh*-Thrombin *in E. coli* werden die in der Literatur beschriebenen Ausbeuten deutlich übertroffen. So liegen die in der Literatur beschriebenen Produktausbeuten an nativem Präthrombin-2 in eukaryotischen Expressionssystemen zwischen 25 und 200mg/l Fermentationsmedium (Russo *et al.* 1997, Yonemura *et al.* 2004). Die erfindungsgemäß erreichten Ausbeuten an nativem Präthrombin-2 liegen dagegen bei 400 mg/l Fermentationsmedium. Es werden Ausbeuten an $\alpha$-Thrombin von 200 mg/l Fermentationsmedium mit einer rp-HPLC Reinheit von mindestens 95 % erreicht. Zudem ist das eingesetzte prokaryotische Expressionssystem einfacher zu etablieren als bekannte eukaryotische Verfahren.

In dem von Soejima *et al.* 2001 beschriebenen prokaryotischen Verfahren werden Faltungsausbeuten an nativem Präthrombin-2 nach Renaturierung von 4 - 7 % erreicht. Im erfindungsgemäß eingereichten Verfahren wurden dagegen 25% Faltungsausbeute erhalten. Außerdem wurde das Volumen des verwendeten Faltungsansatzes verringert (Erhöhung der Proteinkonzentration in der Faltung).

**Patentansprüche**

1. Verfahren zur Herstellung von gefaltetem Präthrombin oder einem Derivat davon, wobei Einschlusskörperchen, die nicht gefaltetes Präthrombin oder ein Derivat davon enthalten, in einem Solubilisierungspuffer solubilisiert werden, der mindestens eine chaotrope Verbindung und mindestens eine organische Disulfidverbindung enthält.

2. Verfahren nach Anspruch 1, wobei die Disulfidverbindung Glutathion-Disulfid ist.

3. Verfahren nach mindestens einem der vorhergehenden Ansprüche, wobei die chaotrope Verbindung ausgewählt ist aus Guanidiniumsalzen, insbesondere Guanidiniumhydrochlorid und Guanidiniumthiocyanat, Jodiden, Bariumsalzen, Thiocyanaten, Harnstoff und Perchloraten.

4. Verfahren nach mindestens einem der vorhergehenden Ansprüche, wobei der Solubilisierungspuffer und/oder Faltungspuffer kein Detergens aufweist.

5. Verfahren nach mindestens einem der vorhergehenden Ansprüche, wobei die Einschlusskörperchen durch rekombinante Expression von Präthrombin oder einem Derivat davon in prokaryotischen Zellen erhalten wurden.

6. Verfahren nach mindestens einem der vorhergehenden Ansprüche, wobei das solubilisierte Präthrombin oder Derivat davon in einem Renaturierungspuffer renaturiert wird, der mindestens ein Reduktionsmittel, mindestens einen Faltungshelfer und divalente Kationen aufweist.

7. Verfahren nach Anspruch 6, wobei das Reduktionsmittel ein organisches Monosulfid ist.

8. Verfahren nach mindestens einem der Ansprüche 6 oder 7, wobei der Faltungshelfer ausgewählt ist aus Arginin und Glycerin.

9. Verfahren nach mindestens einem der Ansprüche 6 bis 8, wobei die Faltung in einem Puls Renaturierungsverfahren durchgeführt wird.

10. Verfahren nach mindestens einem der Ansprüche 6 bis 9, wobei das renaturierte Präthrombin oder Derivat chromatographisch gereinigt wird.

11. Verfahren nach Anspruch 10, wobei die Reinigung durch Hydrophobe Interaktions-Chromatographie (HIC) erfolgt

12. Verfahren zur Herstellung von Thrombin und/oder $\alpha$-Thrombin oder einem Derivat davon, wobei das gemäß einem der Ansprüche 6 bis 11 hergestellte renaturierte Präthrombin durch enzymatische Proteolyse, insbesondere mit Ecarin, zu Thrombin und/oder $\alpha$-Thrombin oder dem Derivat davon umgesetzt wird.

13. Verfahren nach Anspruch 12, wobei das Thrombin und/oder $\alpha$-Thrombin oder Derivat davon nach der Proteolyse chromatographisch gereinigt wird.

14. Verfahren nach Anspruch 13, wobei die Reinigung durch Hydrophobe Interaktions-Chromatographie (HIC) erfolgt.

15. Lösung enthaltend gefaltetes Präthrombin, gefaltetes Thrombin und/oder gefaltetes $\alpha$-Thrombin, oder ein Derivat davon, erhältlich nach einem Verfahren gemäß mindestens einem der vorhergehenden Ansprüche.

Figur 1:

| Spur | Probe |
|------|-------|
| 1 | Mark 12$^{TM}$ |
| 2 | PT-2 nach Renaturierung |

Präthrombin-2

**Figur 2:**

**Figur 3:**

## Sequence Präthrombin 2

*MTATSEYQTFFNPR*TFGSGEADCGLRPLFEKKSLEDKTERELLESYIDGRIVEGSD
AEIGMSPWQVMLFRKSPQELLCGASLISDRWVLTAAHCLLYPPWDKNFT
ENDLLVRIGKHSRTRYERNIEKISMLEKIYIHPRYNWRENLDRDIALMKLK
KPVAFSDYIHPVCLPDRETAASLLQAGYKGRVTGWGNLKETWTANVGK
GQPSVLQVVNLPIVERPVCKDSTRIRITDNMFCAGYKPDEGKRGDACEGD
SGGPFVMKSPFNNRWYQMGIVSWGEGCDRDGKYGFYTHVFRLKKWIQK
VIDQFGE

N-Terminus (fett und kursiv):

Aminosäuren: 14

Molekulargewicht: 1692.8

Theoretical pI: 5.75

A-Kette (grau unterlegt):

Aminosäuren: 36

Molekulargewicht: 4090.5

Theoretischer pI 4.65

B-Chain:

Aminosäuren: 259

Molekulargewicht: 29738.1

Theoretischer pI: 8.88

PT2:

Aminosäuren: 309

Molekulargewicht: 35485.5

Theoretischer pI 8.32

**Figur 4:**

## Sequenz α-Thrombin

TFGSGEADCGLRPLFEKKSLEDKTERELLESYIDGRIVEGSDAEIGMSPWQVMLFRKSP
QELLCGASLISDRWVLTAAHCLLYPPWDKNFTENDLLVRIGKHSRTRYERNIEKISMLEKI
YIHPRYNWRENLDRDIALMKLKKPVAFSDYIHPVCLPDRETAASLLQAGYKGRVTGWGN
LKETWTANVGKGQPSVLQVVNLPIVERPVCKDSTRIRITDNMFCAGYKPDEGKRGDACE
GDSGGPFVMKSPFNNRWYQMGIVSWGEGCDRDGKYGFYTHVFRLKKWIQKVIDQFGE

A-Kette (grau unterlegt):

Aminosäuren: 36

Molekulargewicht: 4090.5

Theoretischer pI 4.65


B-Kette:

Aminosäuren: 259

Molekulargewicht:: 29738.1

Theoretischer pI 8.88


aT:

Aminosäuren: 295

Molekulargewicht: 33810.7

Theoretischer pI 8.32

Europäisches
Patentamt

European
Patent Office

Office européen
des brevets

# EUROPÄISCHER RECHERCHENBERICHT

**Nummer der Anmeldung**

EP 10 16 0740

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 2002 306163 A (CHEMO SERO THERAPEUT RES INST) 22. Oktober 2002 (2002-10-22) | 1-8,10, 12,13,15 | INV. C12N9/74 |
| Y | * das ganze Dokument * ----- | 9,11,14 | C07K1/113 |
| X,D | SOEJIMA K ET AL: "AN EFFICIENT REFOLDING METHOD FOR THE PREPARATION OF RECOMBINANT HUMAN PRETHROMBIN-2 AND CHARACTERIZATION OF THE RECOMBINANT DERIVED ALPHA-THROMBIN" JOURNAL OF BIOCHEMISTRY, JAPANESE BIOCHEMICAL SOCIETY / OUP, TOKYO; JP, Bd. 130, Nr. 2, 1. August 2001 (2001-08-01), Seiten 269-277, XP009041604 ISSN: 0021-924X | 1,3-8, 10,12, 13,15 | |
| Y | * das ganze Dokument * ----- | 2,9,11, 14 | |
| X | DIBELLA ELSIE E ET AL: "Expression and Folding of Recombinant Bovine Prethrombin-2 and Its Activation to Thrombin" JOURNAL OF BIOLOGICAL CHEMISTRY, Bd. 270, Nr. 1, 1995, Seiten 163-169, XP002149152 ISSN: 0021-9258 | 1-5,15 | RECHERCHIERTE SACHGEBIETE (IPC) C12N C07K |
| Y | * das ganze Dokument * ----- | 6-14 | |
| X | WO 00/71692 A1 (MEDICAL RES COUNCIL [GB]; FERSHT ALAN [GB]) 30. November 2000 (2000-11-30) | 15 | |
| Y | * Seite 2, Zeile 5 - Seite 3, Zeile 16 * * Seite 15, Zeile 1 - Seite 22, Zeile 2 * ----- | 1-14 | |
| | -/-- | | |

Der vorliegende Recherchenbericht wurde für alle Patentansprüche erstellt

| Recherchenort | Abschlußdatum der Recherche | Prüfer |
|---|---|---|
| München | 10. November 2010 | Donath, Cornelia |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

| | Europäisches Patentamt European Patent Office Office européen des brevets | **EUROPÄISCHER RECHERCHENBERICHT** | **Nummer der Anmeldung** EP 10 16 0740 |
|---|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WANG FANGWEI ET AL: "Glycerol-assisted hydrophobic interaction chromatography improving refolding of recombinant human granulocyte colony-stimulating factor." APPLIED BIOCHEMISTRY AND BIOTECHNOLOGY DEC 2009 LNKD- PUBMED:19169864, Bd. 159, Nr. 3, Dezember 2009 (2009-12), Seiten 634-641, XP009140886 ISSN: 1559-0291 * das ganze Dokument * ----- | 1-14 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| München | 10. November 2010 | Donath, Cornelia |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
........................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 10 16 0740

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-11-2010

| Im Recherchenbericht angeführtes Patentdokument | Datum der Veröffentlichung | Mitglied(er) der Patentfamilie | Datum der Veröffentlichung |
|---|---|---|---|
| JP 2002306163    A | 22-10-2002 | KEINE | |
| WO 0071692    A1 | 30-11-2000 | AU      5082100 A<br>CA      2370699 A1<br>EP      1194532 A1<br>JP   2003500049 T<br>US   2002142369 A1 | 12-12-2000<br>30-11-2000<br>10-04-2002<br>07-01-2003<br>03-10-2002 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 05061716 A

### In der Beschreibung aufgeführte Nicht-Patentliteratur

- Annual Report on Form 10-K. Zymogenetics, Inc, 31. Dezember 2003
- **NAKAJIMA et al.** *Ann. Thorac. Surg.,* 2005, vol. 79, 1793-1794
- **ESMON ; JACKSON.** *Journal of Biological Chemistry,* 1974, vol. 249 (24), 7791-7797
- **GRAND et al.** *Biochemical Journal,* 1996, vol. 313 (2), 353-368
- **TSOPANOGLOU ; MARAGOUDAKIS.** *Journal of Biological Chemistry,* 1999, vol. 274 (34), 23969-23976
- **JENNY et al.** *Protein Expression and Purification,* 2003, vol. 31 (1), 1-11
- **BODE et al.** *Protein Science,* 1992, vol. 1 (4), 426-471
- **STUBBS ; BODE.** *Thrombosis Research.,* 1993, vol. 69 (1), 1-58
- **STUBBS ; BODE.** *Trends Biochem Sci.,* 1995, vol. 20 (1), 23-8
- *Trends Biochem Sci,* Marz 1995, vol. 20 (3), 131
- **BUTENAS ; MANN.** *Biochemistry,* 2002, vol. 67 (1), 3-12
- **ROSING et al.** *Journal of Biological Chemistry,* 1986, vol. 261, 4224-4228
- **RUSSO et al.** *Protein Expr. Purif.,* 1997, vol. 10 (2), 214-25
- **YONEMURA et al.** *J. Biochem.,* 2004, vol. 135 (5), 577-82
- **SOEJIMA et al.** *Journal of Biochemistry,* 2001, vol. 130 (2), 269-277
- **VIEIRA ; MESSING.** *Gene,* Oktober 1982, vol. 19 (3), 259-68

SEQUENCE LISTING

<110>  Scil Proteins GmbH

<120>  Verfahren zur Herstellung von rekombinantem Thrombin

<130>  P29042

<160>  2

<170>  PatentIn version 3.3

<210>  1
<211>  309
<212>  PRT
<213>  Homo sapiens

<400>  1

```
Met Thr Ala Thr Ser Glu Tyr Gln Thr Phe Phe Asn Pro Arg Thr Phe
1               5                   10                  15

Gly Ser Gly Glu Ala Asp Cys Gly Leu Arg Pro Leu Phe Glu Lys Lys
                20                  25                  30

Ser Leu Glu Asp Lys Thr Glu Arg Glu Leu Leu Glu Ser Tyr Ile Asp
            35                  40                  45

Gly Arg Ile Val Glu Gly Ser Asp Ala Glu Ile Gly Met Ser Pro Trp
        50                  55                  60

Gln Val Met Leu Phe Arg Lys Ser Pro Gln Glu Leu Leu Cys Gly Ala
65                  70                  75                  80

Ser Leu Ile Ser Asp Arg Trp Val Leu Thr Ala Ala His Cys Leu Leu
                85                  90                  95

Tyr Pro Pro Trp Asp Lys Asn Phe Thr Glu Asn Asp Leu Leu Val Arg
                100                 105                 110

Ile Gly Lys His Ser Arg Thr Arg Tyr Glu Arg Asn Ile Glu Lys Ile
                115                 120                 125

Ser Met Leu Glu Lys Ile Tyr Ile His Pro Arg Tyr Asn Trp Arg Glu
        130                 135                 140

Asn Leu Asp Arg Asp Ile Ala Leu Met Lys Leu Lys Lys Pro Val Ala
145                 150                 155                 160

Phe Ser Asp Tyr Ile His Pro Val Cys Leu Pro Asp Arg Glu Thr Ala
                165                 170                 175

Ala Ser Leu Leu Gln Ala Gly Tyr Lys Gly Arg Val Thr Gly Trp Gly
                180                 185                 190

Asn Leu Lys Glu Thr Trp Thr Ala Asn Val Gly Lys Gly Gln Pro Ser
                195                 200                 205
```

```
Val Leu Gln Val Val Asn Leu Pro Ile Val Glu Arg Pro Val Cys Lys
    210             215             220

Asp Ser Thr Arg Ile Arg Ile Thr Asp Asn Met Phe Cys Ala Gly Tyr
225             230             235             240

Lys Pro Asp Glu Gly Lys Arg Gly Asp Ala Cys Glu Gly Asp Ser Gly
            245             250             255

Gly Pro Phe Val Met Lys Ser Pro Phe Asn Asn Arg Trp Tyr Gln Met
            260             265             270

Gly Ile Val Ser Trp Gly Glu Gly Cys Asp Arg Asp Gly Lys Tyr Gly
            275             280             285

Phe Tyr Thr His Val Phe Arg Leu Lys Lys Trp Ile Gln Lys Val Ile
    290             295             300

Asp Gln Phe Gly Glu
305
```

<210> 2
<211> 295
<212> PRT
<213> Homo sapiens

<400> 2

```
Thr Phe Gly Ser Gly Glu Ala Asp Cys Gly Leu Arg Pro Leu Phe Glu
1               5               10              15

Lys Lys Ser Leu Glu Asp Lys Thr Glu Arg Glu Leu Leu Glu Ser Tyr
            20              25              30

Ile Asp Gly Arg Ile Val Glu Gly Ser Asp Ala Glu Ile Gly Met Ser
            35              40              45

Pro Trp Gln Val Met Leu Phe Arg Lys Ser Pro Gln Glu Leu Leu Cys
    50              55              60

Gly Ala Ser Leu Ile Ser Asp Arg Trp Val Leu Thr Ala Ala His Cys
65              70              75              80

Leu Leu Tyr Pro Pro Trp Asp Lys Asn Phe Thr Glu Asn Asp Leu Leu
            85              90              95

Val Arg Ile Gly Lys His Ser Arg Thr Arg Tyr Glu Arg Asn Ile Glu
            100             105             110

Lys Ile Ser Met Leu Glu Lys Ile Tyr Ile His Pro Arg Tyr Asn Trp
            115             120             125

Arg Glu Asn Leu Asp Arg Asp Ile Ala Leu Met Lys Leu Lys Lys Pro
    130             135             140
```

```
Val Ala Phe Ser Asp Tyr Ile His Pro Val Cys Leu Pro Asp Arg Glu
145             150             155             160

Thr Ala Ala Ser Leu Leu Gln Ala Gly Tyr Lys Gly Arg Val Thr Gly
            165             170             175

Trp Gly Asn Leu Lys Glu Thr Trp Thr Ala Asn Val Gly Lys Gly Gln
            180             185             190

Pro Ser Val Leu Gln Val Val Asn Leu Pro Ile Val Glu Arg Pro Val
            195             200             205

Cys Lys Asp Ser Thr Arg Ile Arg Ile Thr Asp Asn Met Phe Cys Ala
    210             215             220

Gly Tyr Lys Pro Asp Glu Gly Lys Arg Gly Asp Ala Cys Glu Gly Asp
225             230             235             240

Ser Gly Gly Pro Phe Val Met Lys Ser Pro Phe Asn Asn Arg Trp Tyr
            245             250             255

Gln Met Gly Ile Val Ser Trp Gly Glu Gly Cys Asp Arg Asp Gly Lys
            260             265             270

Tyr Gly Phe Tyr Thr His Val Phe Arg Leu Lys Lys Trp Ile Gln Lys
            275             280             285

Val Ile Asp Gln Phe Gly Glu
290             295
```